(19) Europäisches Patentamt

European Patent Office

Office européen des brevets

(11) **EP 1 703 272 A1**

(12) **EUROPEAN PATENT APPLICATION**

(43) Date of publication:
   20.09.2006 Bulletin 2006/38

(51) Int Cl.:
   ***G01N 21/35*** *(2006.01)*   ***G01N 33/28*** *(2006.01)*
   ***G01N 21/03*** *(2006.01)*

(21) Application number: **05251580.6**

(22) Date of filing: **16.03.2005**

(84) Designated Contracting States:
   **AT BE BG CH CY CZ DE DK EE ES FI FR GB GR HU IE IS IT LI LT LU MC NL PL PT RO SE SI SK TR**
   Designated Extension States:
   **AL BA HR LV MK YU**

(71) Applicant: **BP Chemicals Limited Sunbury-on-Thames, Middlesex TW16 7BP (GB)**

(72) Inventor: **The designation of the inventor has not yet been filed**

(74) Representative: **De Kezel, Eric et al BP International Limited, Group Patents and Agreements, Research and Engineering Centre, Chertsey Road Sunbury-on-Thames, Middlesex TW16 7LN (GB)**

Remarks:
A request for correction of the title has been filed pursuant to Rule 88 EPC. A decision on the request will be taken during the proceedings before the Examining Division (Guidelines for Examination in the EPO, A-V, 3.).

(54) **Measuring near infra-red spectra using a demountable NIR transmission cell**

(57) The present invention provides a process for measuring the NR spectrum of a sample using a demountable NIR transmission cell of pathlength of 2.5mm or less, said process comprising:

(a) measuring the etalon fringes that arise when NIR light passes through the NIR cell in the absence of a liquid sample,
(b) using this to calculate the pathlength of the NIR cell,
(c) introducing the sample to be analysed in to the NIR cell, and
(d) measuring the NIR spectrum of the sample.

EP 1 703 272 A1

**Description**

The present invention relates to the measurement of near infra-red (NIR) spectra, and, in particular, to measurement of NIR spectra using a demountable NIR transmission cell.

NIR spectroscopy is a well-known spectroscopic technique. NIR looks at absorptions of infra-red radiation with wavelengths above 4000cm$^{-1}$. NIR spectroscopy can be used to measure the intensity of the overtones of the molecular vibrations in a molecule, containing carbon-hydrogen, oxygen-hydrogen, and nitrogen-hydrogen bonds. The carbon-hydrogen (C-H) absorption bands are typically useful for mixtures of organic compounds. Different types of C-H bonds, e.g., aromatic, aliphatic, and olefinic hydrocarbons, absorb light at different characteristic frequencies. The magnitude of the absorption band is proportional to the amounts of the C-H bonds in the sample. Therefore the NIR spectrum can provide a fingerprint of the sample composition. This fingerprint can be empirically correlated to the intrinsic properties of the sample.

The NIR region between 780 nanometers (nm) and 2500 nm (12800 to 4000 cm$^1$) contains a large amount of molecular information in the form of combinations and overtones from polyatomic vibrations, and mathematical techniques are needed to utilize this information and to calculate the desired properties. U.S. Patent Nos. 5,490,085; 5,452,232; and 5,475,612, for example, describe the use of NIR for determining octane number, yields and/or properties of a product of a chemical process or separation process from analysis on the feeds to that process, and yields and/or properties of a product of a blending operation again from analysis on the feed thereto.

NIR spectroscopy can be applied to crude oils and other hydrocarbon refinery streams. WO 00/039561 and WO 03/048759, for example, both describe application of NIR to crude oil analysis.

The analysis of crude oil samples, for example, can be performed by generating chemometric models correlating spectral data from "standard" (i.e. characterised) crude oil samples with the known properties of the samples, and subsequently applying said models to the spectra of "unknown" samples to characterise the properties thereof.

The analysis of crude oils is typically done using a transmission cell into which the sample of crude oil is introduced. The cells typically have a relatively short pathlength so that a reasonable signal is transmitted through the cell. However, such cells require cleaning when used with crude oils. This is not trivial with fixed (solid) cells, so demountable cells are preferred. Demountable cells may be taken apart, cleaned and then put back together again for re-use.

One problem with demountable cells, however, is that during the disassembly and reassembly the pathlength of the cell may change. With cells that have a relatively short pathlength, even small changes in the pathlength can have significant effects on the spectra obtained.

Where spectral data from "standard" (i.e. characterised) crude oil samples is being measured for generation of a suitable chemometric model correlating various properties of the crude oil samples with the spectral data, the variations in cell pathlength can have significant effects on the accuracy of the model obtained.

We have now found an improved method of measuring the pathlength, during measurement of samples, such as crude oils, in a demountable NIR transmission cell. This method can also be used to generate improved chemometric models, which can themselves be applied for identification of the properties of unknown samples.

Thus, in a first aspect, the present invention provides a process for measuring the NIR spectrum of a sample using a demountable NIR transmission cell of pathlength of 2.5mm or less, said process comprising:

(a) measuring the etalon fringes that arise when NIR light passes through the NIR cell in the absence of a liquid sample,
(b) using this to calculate the pathlength of the NIR cell,
(c) introducing the sample to be analysed in to the NIR cell, and
(d) measuring the NIR spectrum of the sample.

The etalon fringes arise from interaction of NIR light that passes straight through the cell with light that bounces off of the inner surfaces of the cell. The etalons are strongest when the difference in the refractive indices of the windows and whatever is within the cell are greatest, and, hence, generally disappear when liquid samples are injected into the cell (the refractive index of liquids generally being similar to that of the cell windows).

The spacing of the etalon fringes is related to the pathlength of the cell (the spacing gets smaller as the pathlength increases), and hence can be used to accurately measure the cell pathlength. Because of the inverse relationship between the spacing and the pathlength, however, the technique is generally most applicable for pathlengths of 2.5mm or less. The preferred "nominal" pathlength of the NIR cell is 1.0mm or less, for example, 1.0mm or 0.5mm.

Although the pathlength of a cell may be measured by filling the cell with a standard solution, such as toluene, and measuring the spectrum, the use of the etalon fringes has been found to provide a rapid and accurate measurement of the cell pathlength, which can be simpler and more accurate than using standard solutions. The process of the present invention may be used for any suitable sample in a demountable cell, but is especially useful for samples that require disassembly and reassembly of the cell for cleaning, and, hence, where the cell is reassembled the most times.

The sample is preferably a liquid sample.

The sample is preferably a hydrocarbon sample, and most preferably a crude oil or "equivalent" sample. By "equivalent" sample is meant a sample that may be used either in place of or blended with a crude oil in a refinery, such as a synthetic crude, a biocomponent, an intermediate stream, such as a residue, gas oil, vacuum gas oil, naphtha or cracked stock, and blends of one or more of said components.

Other hydrocarbon samples to which the process of the present invention may be applied include fuels, lubricants, polymers (liquid polymers or polymer melts) and petrochemicals which are prone to fouling.

The NIR cell may have windows of any suitable material, such as silica, sapphire or standard salt windows (such as CaF2, MgF2, NaCl, KBr).

The etalon fringes in step (a) are measured by measuring a background spectrum in the assembled cell with air (or other gas or vacuum if appropriate) in the cell.

The pathlength of the cell may be calculated from the spacing of the fringes, and the pathlength (in mm) is equal to five divided by the spacing of adjacent fringes (in wavenumbers). The accuracy of the measurement may be increased by measuring the average spacing over a number of etalon fringes, typically over 10 fringes or greater. The pathlength may thus be calculated according to the general formula given by equation 1:

$$\text{Pathlength (mm)} = [5 \times n] / [V(1) - V(n)] \qquad \text{Equation 1}$$

Where:

n is the number of fringes within the wavenumber range from V(1) to V(n),
V(1) is the wavenumber of the first fringe, and
V(n) is the wavenumber of the $n^{th}$ fringe.

The measurement of the sample is then performed, to give a spectrum of absorbance bands characteristic of the sample.

Preferably, the sample is a hydrocarbon sample, and preferably a crude oil or "equivalent" sample, which has known chemical and/or physical properties, and the spectral NIR data produced and corresponding pathlength of the cell measured is used as an input to a chemometric model correlating the spectral data with the known properties and the measured pathlength of the cell.

For example, for a crude oil sample, known chemical and/or physical properties may include one or more of true boiling profile (TBP), API gravity, viscosity, cold flow properties and molecular composition, such as sulphur content, benzene content and aromatics content.

"Chemometrics" is the application of mathematical and statistical techniques to the analysis of complex data, and hence "chemometric model" as used herein means a model generated from application of such techniques in correlating the spectral data from a sample with properties of the sample and cell pathlength. The chemometric model determines the relationship between the spectral data and the cell pathlength as it would for the chemical and/or physical properties (via eigenvectors of a covariance matrix).

Thus, in a second aspect, the present invention provides a process for generating a chemometric model correlating NIR spectral data with physical and/or chemical properties of a sample set and measured pathlength of the NIR cell, using a plurality of samples within the sample set with known physical and/or chemical properties and a demountable NIR transmission cell of pathlength of 2.5mm or less, said process comprising:

(a) measuring the etalon fringes that arise when NIR light passes through the NIR cell in the absence of a liquid sample,
(b) using this to calculate the pathlength of the NIR cell,
(c) introducing a first sample to be analysed in to the NIR cell,
(d) measuring the NIR spectrum of the first sample,
(e) repeating steps (a) to (d) for each of the plurality of samples in the sample set, and
(f) chemometrically analysing the NIR spectral data obtained, the respective pathlength measurements and the physical and/or chemical properties of each sample in the sample set to generate the chemometric model.

The samples and demountable NIR cell are preferably as described for the first aspect.

"Sample set" as used herein refers to the class of sample, such as crude oils generally. Thus, a plurality of crude oils may be used to generate a chemometric model correlating properties of crude oils with the spectral data.

Preferably, the plurality of samples with known physical and/or chemical properties comprises at least 20 samples, more preferably at least 50 samples and most preferably at least 100 samples.

Preferably, in steps (a) and (d) of the second aspect, the respective NIR measurements are measured at the same temperature for each of the plurality of samples so that temperature effects are minimised. Suitably, the temperature is in the range 10°C to 100°C. For practical purposes a temperature slightly above ambient temperature, for example, in the range 30°C to 60°C is most preferred.

The generation of the chemometric model may be done using any suitable chemometric techniques/mathematical and statistical techniques, as described, for example, in Principal Component Analysis, I. T. Jolliffe, Springer-Verlag, New York, 1986; D. M. Halland and E. V. Thomas, Anal. Chem., 60, 1202 (1988) or K. R. Beebe and B. R. Kowalski, Anal. Chem., 59, 1007A (1987).

The chemometric model of the second aspect has the advantage that the cell pathlength is treated as any other property of the sample. Thus, the model is robust to variations of pathlength in the measurement of NIR spectra of unknown samples for subsequent determination of the properties of the unknown samples.

Thus, in a third aspect, the present invention provides a process for the analysis of a sample, which process comprises:

(i) introducing the sample to be analysed in to an NIR transmission cell of pathlength of 2.5mm or less,
(ii) measuring the NIR spectrum of the sample, and
(iii) applying a chemometric model generated according to the second aspect of the invention to determine physical and/or chemical properties of the sample.

The third aspect of the present invention has the advantage that it is not necessary to measure or input into the chemometric model the cell pathlength used for the "unknown" sample to be analysed. Instead the model scales the spectrum of the sample based on the mean pathlength of the sample set used in the second aspect to generate the chemometric model.

The process of the third aspect of the present invention allows physical and/or chemical properties of a number of samples to be determined from the NIR spectra thereof, even though the pathlength of the cell may vary between samples, because the pathlength is treated as another property in the chemometric model.

In one embodiment, this variation may be due to the use of a demountable cell which is disassembled and reassembled between samples.

In a second embodiment, physical and/or chemical properties of samples may be determined from NIR spectra in a number of separate demountable cells, for example, spectra obtained in different laboratories may be compared. This is particularly useful where NIR spectra of samples measured in multiple different cells need to be compared, as the tolerance limits for each cell (i.e. how close the actual cell pathlength need be to a nominal pathlength) can be relaxed because the process of the present invention can adjust the data for the actual pathlength.

Finally, it is also possible to use NIR cells which do not have a constant pathlength, by which is meant that all light that passes into the cell does not pass through the same pathlength of sample, such as a cylindrical cell. In this case, the chemometric model allows for this by correlating to the average pathlength that light actually travels through the NIR cell.

Example

1) Generation of calibration data.
0.5, 1.0 and 2.0mm pathlength NIR cells were filled with toluene and the absorption area of the first overtone band of CH at 5951cm$^{-1}$ was measured. The measured data was fitted to a straight line of area versus cell pathlength, giving a gradient of 112.84 and an $R^2$ of 0.9999 (indicating a very good fit).

2) Measurements of empty and toluene-filled NIR cell.
The NIR cell was then disassembled and reassembled 6 times, each time a measurement of the empty cell (air filled) and the cell filled with toluene being made by NIR.

3) Measurements of toluene spectra.
The measured spectra of toluene are given in Table 1.

Table 1

| Measurement | Area at 5951 cm$^{-1}$ | Calculated pathlength (mm) |
|---|---|---|
| 1a | 56.2892 | 0.4988 |
| 2a | 56.3318 | 0.4992 |
| 3a | 56.4182 | 0.5000 |

(continued)

| Measurement | Area at 5951 cm$^{-1}$ | Calculated pathlength (mm) |
|---|---|---|
| 4a | 56.5772 | 0.5014 |
| 5a | 56.3047 | 0.4990 |
| 6a | 56.4716 | 0.5005 |

Mean pathlength = 0.4998
Standard deviation = 0.0010
4) Use of etalon fringes
The etalon fringes between approx. 6195 and 5855 measured in the empty cells in (1) were used to calculate the pathlength. The results are shown in Table 2.

Table 2

| Measurement | V(1) (cm$^{-1}$) | V(n) (cm$^{-1}$) | No. of fringes (n) | Calculated pathlength (mm) |
|---|---|---|---|---|
| 1b | 6196.28 | 5859.16 | 34 | 0.5043 |
| 2b | 6191.58 | 5854.89 | 34 | 0.5049 |
| 3b | 6191.65 | 5854.93 | 34 | 0.5049 |
| 4b | 6194.06 | 5856.87 | 34 | 0.5042 |
| 5b | 6196.57 | 5858.82 | 34 | 0.5033 |
| 6b | 6195.11 | 5857.87 | 34 | 0.5041 |

Mean pathlength = 0.5043
Standard deviation = 0.0006

It can be seen that the standard deviation of the measurements made using the etalon fringes is significantly lower than that using toluene as a standard, hence providing a more accurate measurement of the pathlength than the toluene method.

**Claims**

1. A process for measuring the NIR spectrum of a sample using a demountable NIR transmission cell of pathlength of 2.5mm or less, said process comprising:

   (a) measuring the etalon fringes that arise when NIR light passes through the NIR cell in the absence of a liquid sample,
   (b) using this to calculate the pathlength of the NIR cell,
   (c) introducing the sample to be analysed in to the NIR cell, and
   (d) measuring the NIR spectrum of the sample.

2. A process as claimed in claim 1 wherein the sample is a crude oil or equivalent sample.

3. A process for generating a chemometric model correlating NIR spectral data with physical and/or chemical properties of a sample set and measured pathlength of the NIR cell, using a plurality of samples within the sample set with known physical and/or chemical properties and a demountable NIR transmission cell of pathlength of 2.5mm or less, said process comprising:

   (a) measuring the etalon fringes that arise when NIR light passes through the NIR cell in the absence of a liquid sample,
   (b) using this to calculate the pathlength of the NIR cell,
   (c) introducing a first sample to be analysed in to the NIR cell,
   (d) measuring the NIR spectrum of the first sample,
   (e) repeating steps (a) to (d) for each of the plurality of samples in the sample set, and

(f) chemometrically analysing the NIR spectral data obtained, the respective pathlength measurements and the physical and/or chemical properties of each sample in the sample set to generate the chemometric model.

4. A process as claimed in claim 3 wherein each of the samples is a crude oil or equivalent sample.

5. A process as claimed in claim 4 wherein the physical and/or chemical properties include one or more of true boiling profile (TBP), API gravity, viscosity, cold flow properties and molecular composition.

6. A process as claimed in any one of claims 3 to 5 wherein the plurality of samples with known physical and/or chemical properties comprises at least 50 samples.

7. A process as claimed in any one of claims 3 to 6 wherein the respective NIR measurements in steps (a) and (d) are measured at the same temperature and at a temperature in the range 30 to 60°C.

8. A process for the analysis of a sample, which process comprises:

(i) introducing the sample to be analysed in to an NIR transmission cell of pathlength of 2.5mm or less,
(ii) measuring the NIR spectrum of the sample, and
(iii) applying a chemometric model generated according to any one of claims 3 to 7 to determine physical and/or chemical properties of the sample.

**EP 1 703 272 A1**

**European Patent Office**

**EUROPEAN SEARCH REPORT**

Application Number

EP 05 25 1580

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARTIN W B ET AL: "Using two discrete frequencies within the middle infrared to quantitatively determine glucose in serum" JOURNAL OF BIOMEDICAL OPTICS, SPIE, BELLINGHAM, WA, US, vol. 7, no. 4, October 2002 (2002-10), pages 613-617, XP002324543 ISSN: 1083-3668 | 1,2 | G01N21/35 G01N33/28 G01N21/03 |
| Y | * page 613, left-hand column, paragraph 3 - right-hand column, paragraph 1 * * page 615, left-hand column, paragraph 2 - right-hand column, paragraph 2 * ----- | 3-8 | |
| Y | WO 92/07275 A (EXXON RESEARCH AND ENGINEERING COMPANY) 30 April 1992 (1992-04-30) | 3-8 | |
| A | * page 1, paragraph 1 * * page 2, paragraphs 2,3 * * page 3, paragraph 2 * ----- | 1 | |
| D,A | WO 03/048759 A (EXXONMOBIL RESEARCH AND ENGINEERING COMPANY) 12 June 2003 (2003-06-12) * the whole document * ----- | 1-8 | TECHNICAL FIELDS SEARCHED (IPC) G01N G01B |
| X | BERTIE J E ET AL: "Determination and use of secondary infrared intensity standards" APPLIED SPECTROSCOPY SOC. APPL. SPECTROSC USA, vol. 49, no. 12, December 1995 (1995-12), pages 1821-1825, XP008057725 ISSN: 0003-7028 * page 1823, left-hand column, paragraph 2 * * tables II,V * ----- | 1,2 | |

The present search report has been drawn up for all claims

| Place of search | Date of completion of the search | Examiner |
|---|---|---|
| The Hague | 22 December 2005 | Verdoodt, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 703 272 A1**

## ANNEX TO THE EUROPEAN SEARCH REPORT
## ON EUROPEAN PATENT APPLICATION NO.

EP 05 25 1580

This annex lists the patent family members relating to the patent documents cited in the above-mentioned European search report.
The members are as contained in the European Patent Office EDP file on
The European Patent Office is in no way liable for these particulars which are merely given for the purpose of information.

22-12-2005

| Patent document cited in search report | Publication date | Patent family member(s) | | Publication date |
|---|---|---|---|---|
| WO 9207275 A | 30-04-1992 | CA | 2092713 A1 | 13-04-1992 |
| | | DE | 69129751 D1 | 13-08-1998 |
| | | DE | 69129751 T2 | 11-02-1999 |
| | | EP | 0552300 A1 | 28-07-1993 |
| | | JP | 3245157 B2 | 07-01-2002 |
| | | JP | 6502492 T | 17-03-1994 |
| | | SG | 48112 A1 | 17-04-1998 |
| WO 03048759 A | 12-06-2003 | AU | 2002348087 A1 | 17-06-2003 |
| | | CA | 2467147 A1 | 12-06-2003 |
| | | EP | 1463937 A1 | 06-10-2004 |
| | | JP | 2005512051 T | 28-04-2005 |
| | | US | 2003195708 A1 | 16-10-2003 |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5490085 A
- US 5452232 A
- US 5475612 A

- WO 00039561 A
- WO 03048759 A

**Non-patent literature cited in the description**

- **I. T. JOLLIFFE.** Principal Component Analysis. Springer-Verlag, 1986
- **D. M. HALLAND ; E. V. THOMAS.** *Anal. Chem.,* 1988, vol. 60, 1202

- **B. R. KOWALSKI.** *Anal. Chem.,* 1987, vol. 59, 1007A